# EUROPEAN PATENT APPLICATION

(11) **EP 4 470 450 A1**
(43) Date of publication of application: **04.12.2024**
(21) Application number: 24178262.2
(22) Date of filing: 27.05.2024
(51) Int. Cl.: A61B 3/11

(54) **OPHTHALMOLOGIC APPARATUS**

(30) Priority: 02.06.2023 JP 2023091738
(71) Applicant: Topcon Corporation, Tokyo 174-8580 (JP)
(72) Inventor: TAKADA, Rei, Tokyo, 174-8580 (JP)
(74) Representative: Louis Pöhlau Lohrentz

(57) **Abstract**

An ophthalmologic apparatus (A) includes an anterior-ocular-segment camera (22, 22', 22a, 22b, 22c) that captures images of an anterior ocular segment (Ea) of a subject eye (E); a trained-model setting unit (621) that is configured to set a trained model for the images of the anterior ocular segment (Ea) of the subject eye (E); and a pupil detection processing unit (632) that is configured to detect a pupil region (PA) of the subject eye (E). The trained-model setting unit (621) is configured to set a trained pupil-region-prediction model that is created by a training process in which a large number of teacher data are prepared by adding a pupil region information to anterior-ocular-segment camera image data collected in advance, and in which the teacher data are read into a selected machine learning model (80). The pupil detection processing unit (632) is configured to detect the pupil region (PA) of the subject eye (E), based on a pupil-region prediction information as a model output (86) that is obtained by an inference operation in which an anterior-ocular-segment camera image data (81) captured by the anterior-ocular-segment camera (22, 22', 22a, 22b, 22c) is input to the trained pupil-region-prediction model.

## Description

### FIELD OF THE INVENTION

The present disclosure relates to an ophthalmologic apparatus.

### BACKGROUND

Hitherto, there has been known an ophthalmologic apparatus in which a stereo camera as an anterior-ocular-segment camera is disposed on both side positions of an objective lens to photograph an anterior ocular segm ent of a subject eye at the same time from different directions, for the purpose of providing an ophthalmologic apparatus that is capable of suitably conducting the positioning between the subject eye and an optical system of the apparatus (see JP2013-248376A).

By the way, when conducting an ocular characteristics examination or photographing of a subject eye with an examinee's chin supported by a chin support, the position of the subject eye shifts with each change of examinees. Therefore, it is necessary to conduct an alignment control (positioning control) to adjust a relative positional relationship between the subject eye and a body portion (= an optical system of the apparatus).

However, stereo camera images captured by stereo cameras are prone to vignetting, in which the pupil is hidden due to the reflection of eyelids and eyelashes. In particular, in the case of ophthalmologic apparatuses such as fundus cameras with a wide angle of view, the lens diameter of the objective lens becomes larger due to optical limitations accompanied by a trend toward a wider angle of view. Thus, the camera mounting angle becomes more acute than the camera mounting angle when the lens diameter is smaller. This results in an elongated oval shape of the pupil of the subject eye, and there is more likely to occur vignetting in which the pupil is hidden by the reflection of eyelids, eyelashes, etc.

In contrast, in a conventional pupil region detection method, it searches for a bright spot in a stereo camera image, the entire image if the bright spot is not detected, or an image around the bright spot if the bright spot is detected, is subjected to a binarization by using a luminance threshold to identify the pupil with the lowest luminance in the image, and then the pupil is detected from the binarized image. Therefore, in the case of the conventional technology to detect a pupil region by using a stereo camera image, if the reflection of eyelids or eyelashes occurs, the binarization produces an image with a pupil region shape in which a part of the pupil region is missing. In this way, if the pupil image has a part with a luminance level higher than the pupil luminance, such high luminance part is not recognized as being the pupil region. As a result, the recognized pupil region lowers in roundness and region area, thereby making it difficult to accurately and stably detect the pupil region.

The present disclosure has been made by considering the above problem. An object of the present disclosure is to provide an ophthalmologic apparatus that can robustly detect a pupil region by an AI image recognition using a trained model at the pupil detection.

### SUMMARY

To achieve the object, an ophthalmologic apparatus includes an anterior-ocular-segment camera that captures images of an anterior ocular segment of a subject eye; a trained-model setting unit that is configured to set a trained model for the images of the anterior ocular segment of the subject eye; and a pupil detection processing unit that is configured to detect a pupil region of the subject eye. The trained-model setting unit is configured to set a trained pupil-region-prediction model that is created by a training process in which a large number of teacher data are prepared by adding a pupil region information to anterior-ocular-segment camera image data collected in advance, and in which the teacher data are read into a selected machine learning model. The pupil detection processing unit is configured to detect the pupil region of the subject eye, based on a pupil-region prediction information as a model output that is obtained by an inference operation in which an anterior-ocular-segment camera image data captured by the anterior-ocular-segment camera is input to the trained pupil-region-prediction model.

The ophthalmologic apparatus of the present disclosure is that the pupil region detection information does not derive from the anterior-ocular-segment camera image information that is affected by pupil detection obstructions such as reflection, but from a pupil region prediction information that is obtained as a model output from a trained pupil-region-prediction model by an inference operation. Therefore, it is possible to robustly detect a pupil region by an AI image recognition using a trained model at the pupil detection.

### BRIEF DESCRIPTION OF DRAWINGS

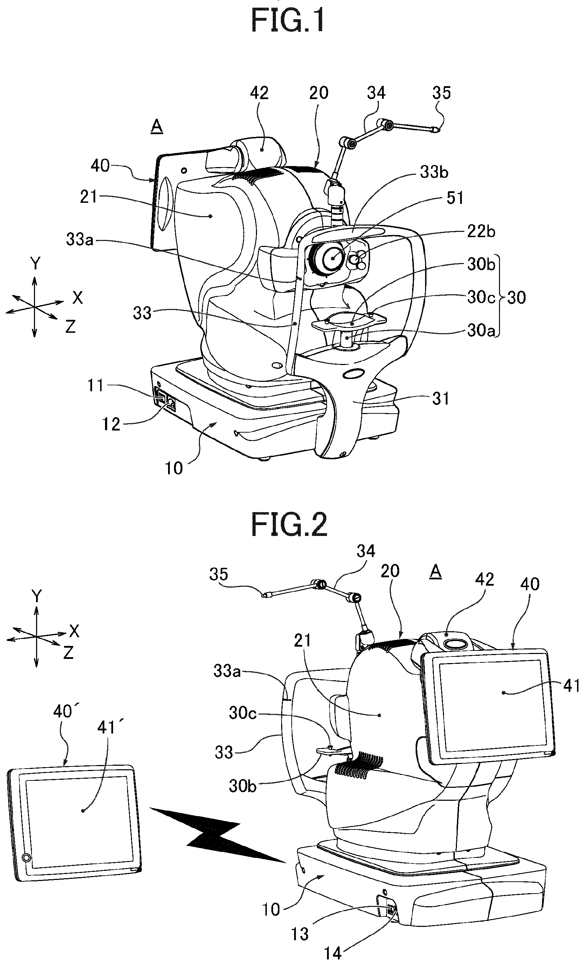

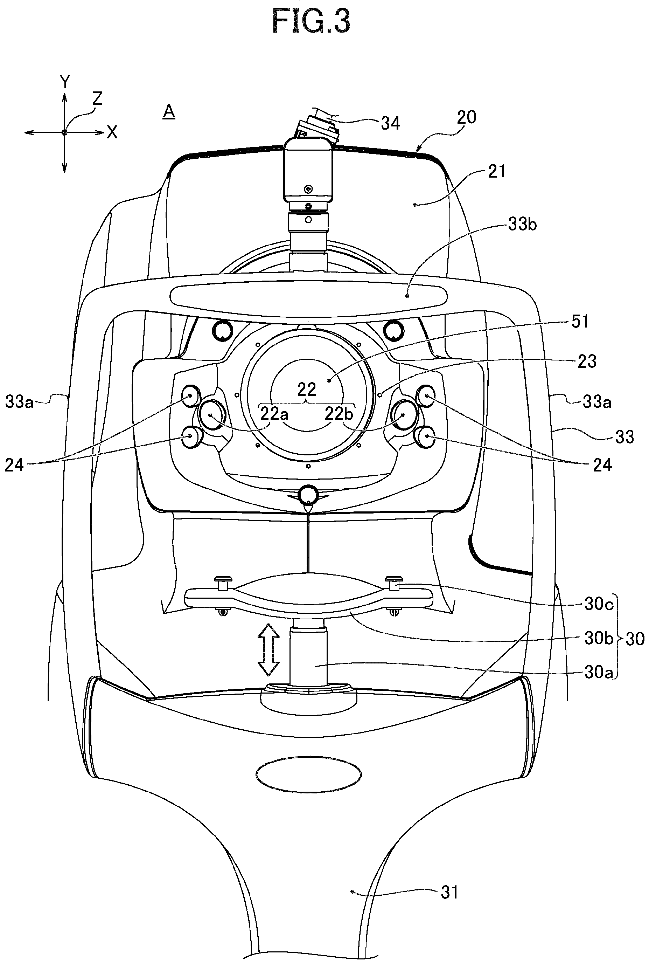

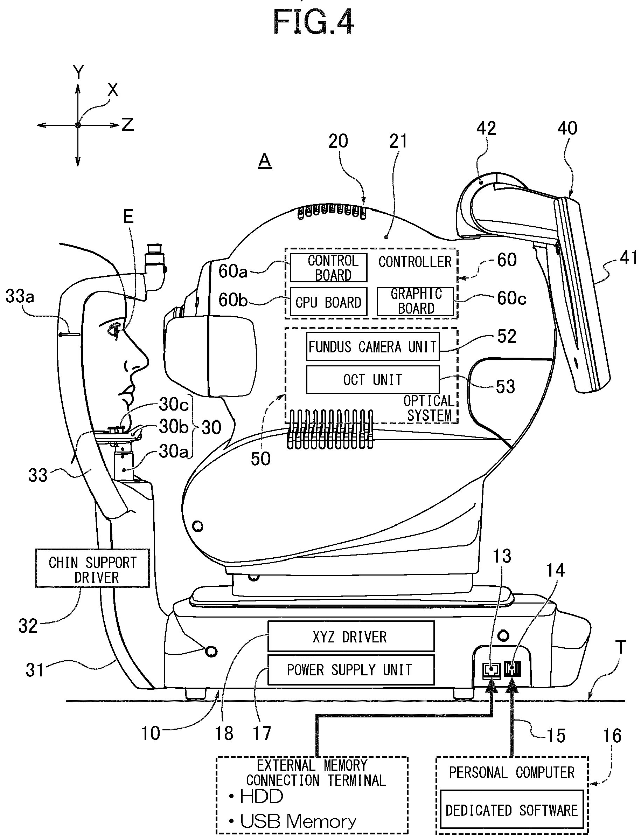

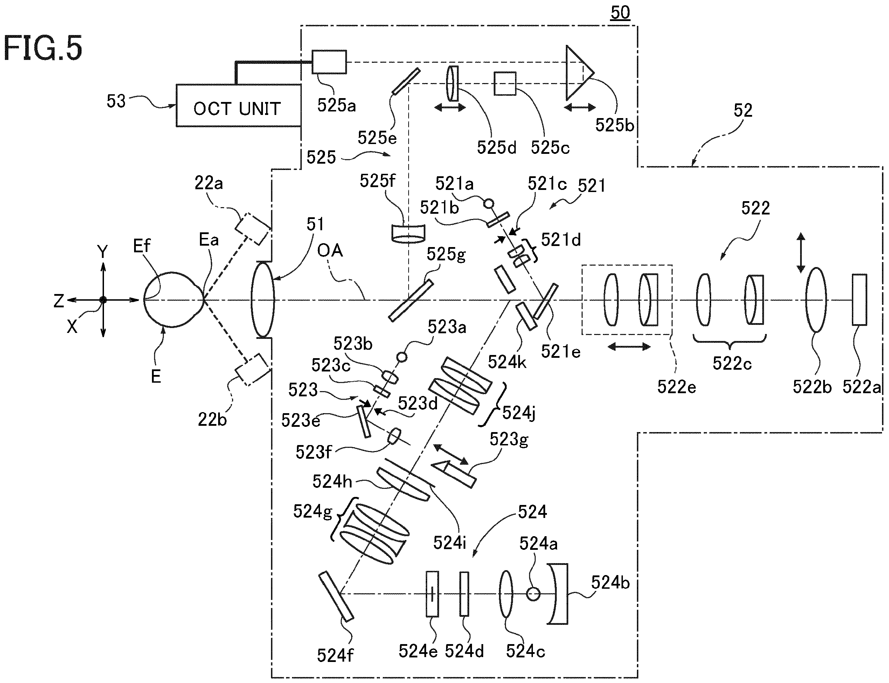

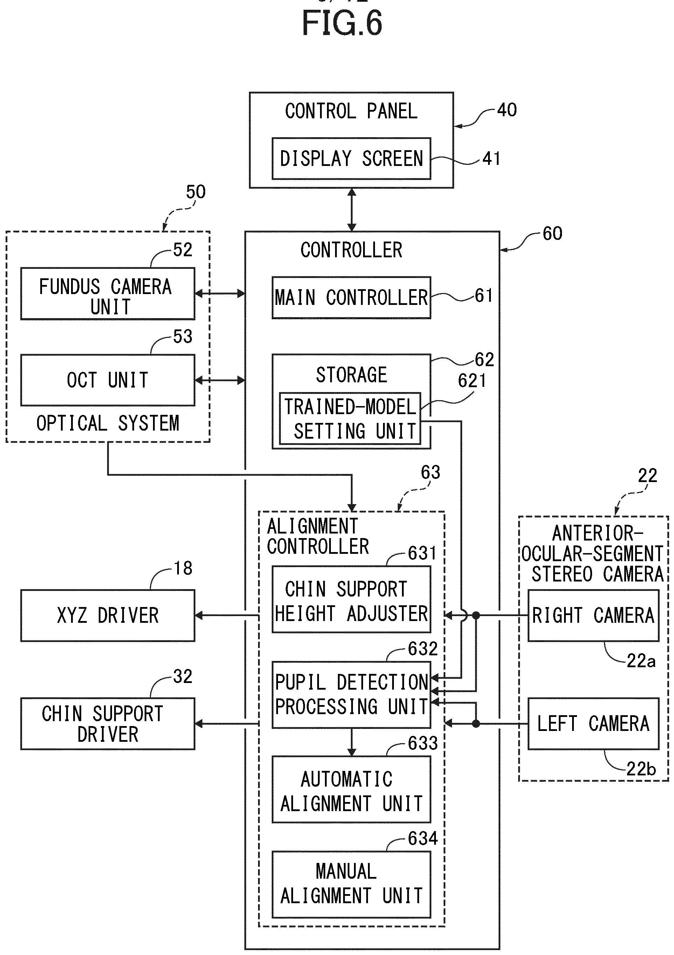

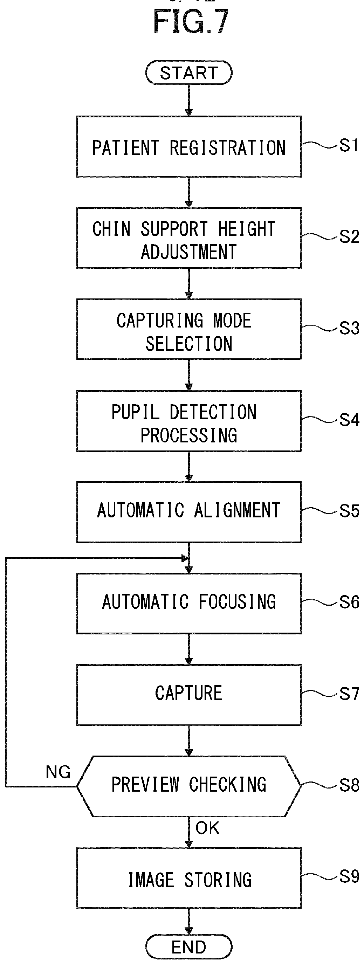

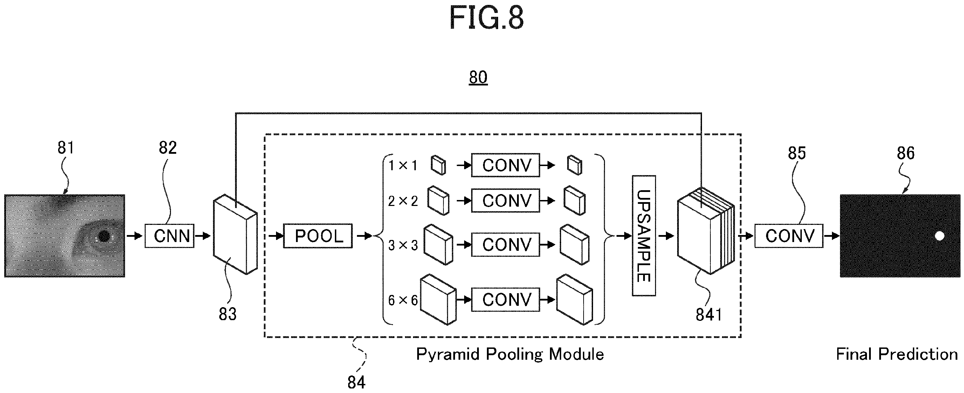

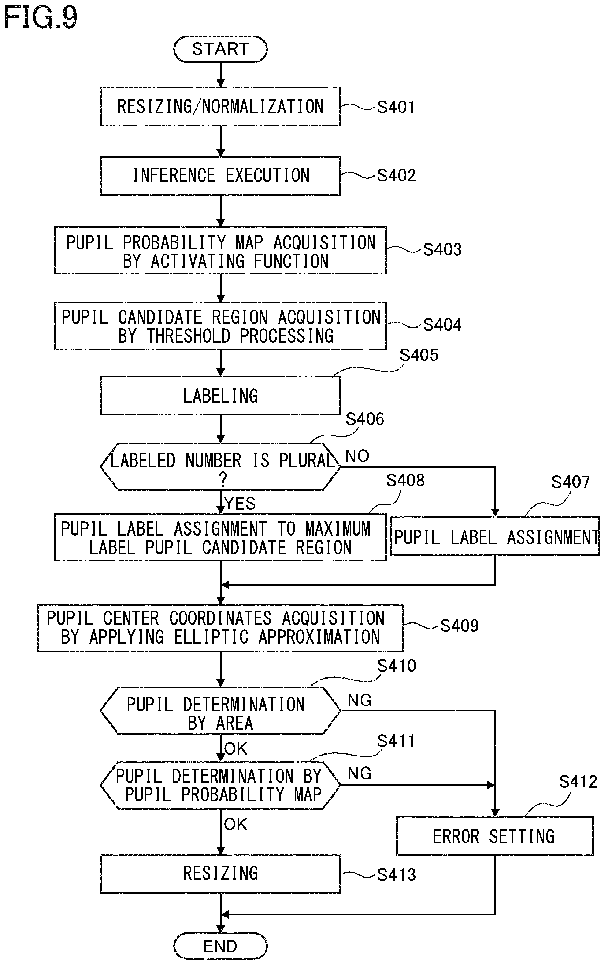

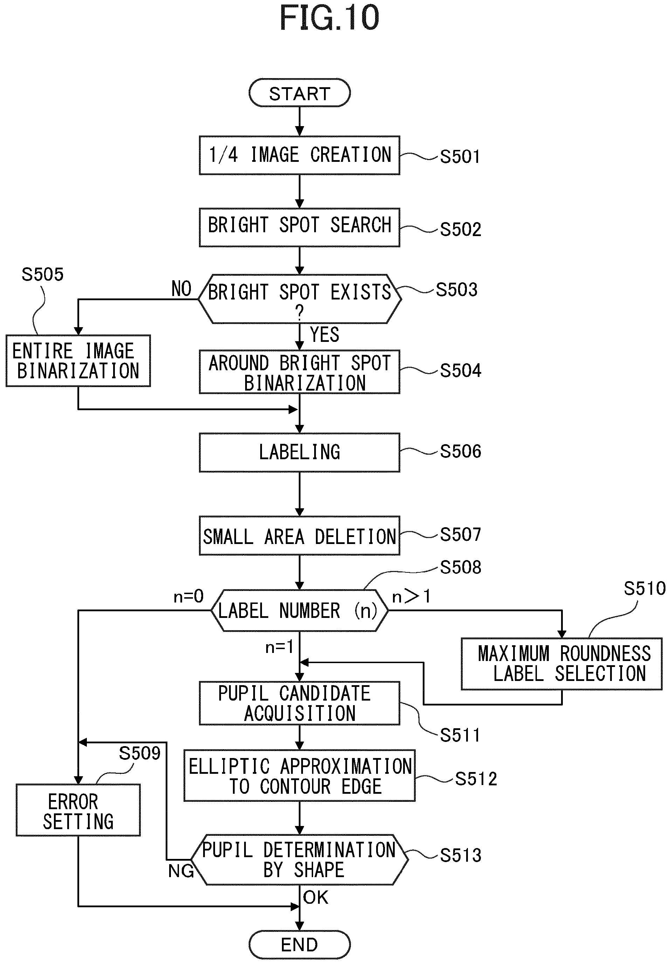

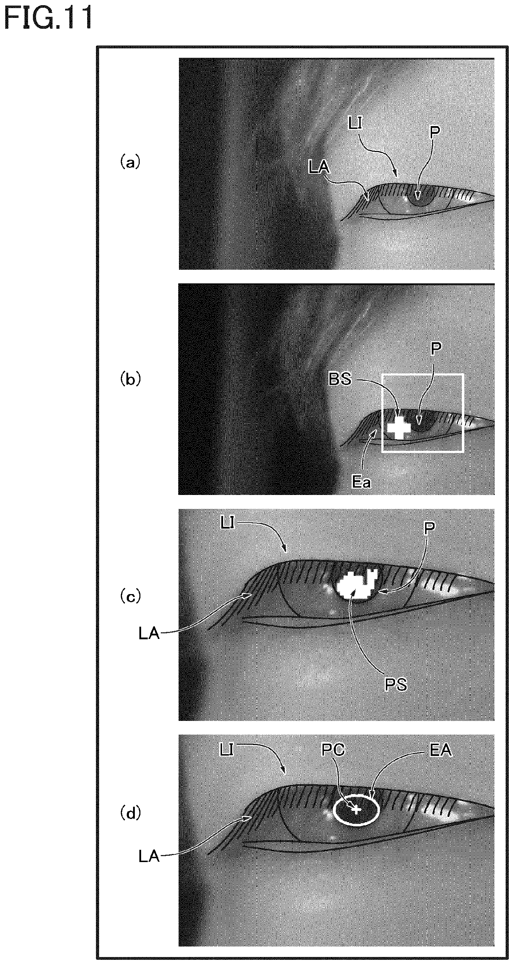

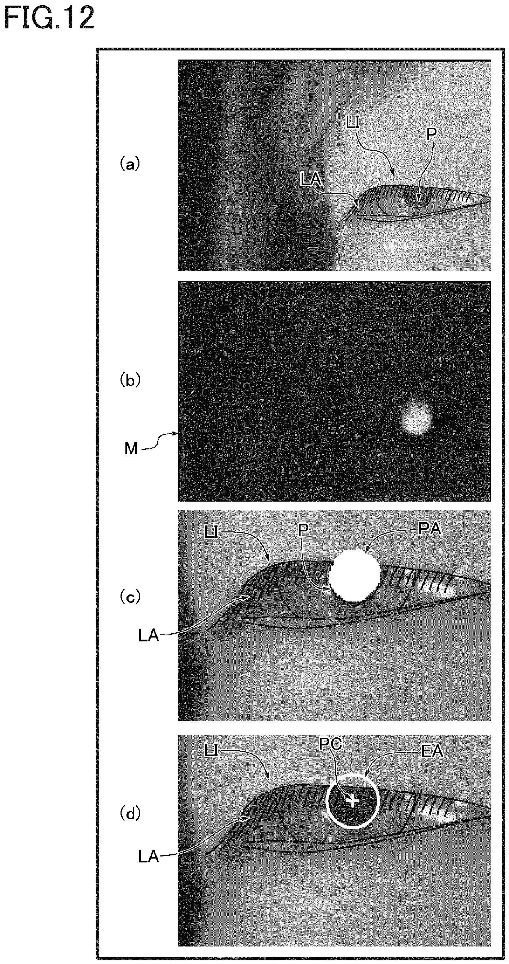

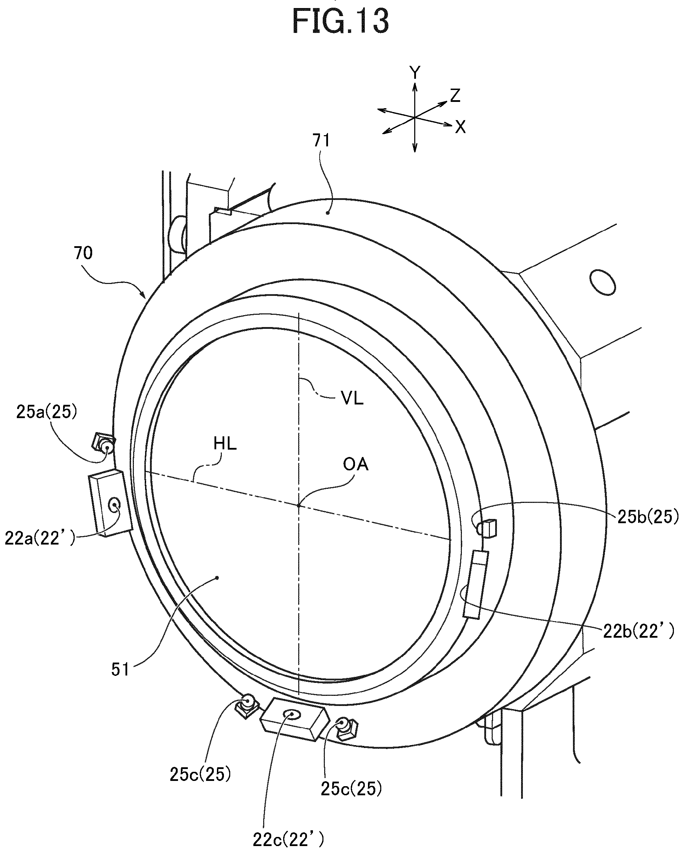

FIG. 1 is a perspective view showing an external appearance of an ophthalmologic apparatus according to a first embodiment, viewed from a chin support side. FIG. 2 is a view similar to FIG. 1, but viewed from a control panel side. FIG. 3 is a front view showing a body portion of the ophthalmologic apparatus according to the first embodiment, viewed from the chin support side. FIG. 4 is a side view showing a schematic configuration of built-in articles and accessories of the ophthalmologic apparatus according to the first embodiment. FIG. 5 is a schematic view showing an optical system configuration in the ophthalmologic apparatus according to the first embodiment. FIG. 6 is a block diagram showing a control system configuration in the ophthalmologic apparatus according to the first embodiment. FIG. 7 is a flowchart showing a basic operation flow, when capturing any of anterior-ocular-segment images, fundus images, and fundus tomographic images of subject eyes by the ophthalmologic apparatus according to the first embodiment. FIG. 8 is a schematic view showing a PSPNet structure selected as a machine learning model in a pupil detection process in a pupil detection processing unit according to the first embodiment. FIG. 9 is a flowchart showing a flow of the pupil detection process in the pupil detection processing unit according to the first embodiment. FIG. 10 is a flowchart showing a flow of the pupil detection process to detect a pupil of a subject eye, prior to an automatic alignment to the pupil in a background technology. FIG. 11 shows image explanatory views in which (a) is an example of anterior-ocular-segment camera image in a pupil detection process of the background technology, (b) is an example of bright spot search from the anterior-ocular-segment image, (c) is an example of binarization around the bright spot, and (d) is an example of pupil center coordinates obtained by applying an elliptic approximation to a contour edge. FIG. 12 shows image explanatory views in which (a) is an example of anterior-ocular-segment camera image in a pupil detection process according to the first embodiment, (b) is an example of pupil probability map, (c) is an example of pupil region with a pupil label in pupil candidate regions, and (d) is an example of pupil center coordinates obtained by applying an elliptic approximation. FIG. 13 is a perspective view showing an objective lens unit, to which an anterior-ocular-segment three-way camera with three cameras is attached, in the ophthalmologic apparatus according to a second embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

The mode for implementing the ophthalmologic apparatus according to the present disclosure is explained based on the first and second embodiments shown in the drawings. The first and second embodiments are examples applied to an ophthalmologic apparatus that observes, photographs and records anterior-ocular-segment images, fundus images, and fundus tomographic images of subject eyes, and provides them as electronic images for diagnosis. In each drawing, when facing the body portion of the ophthalmologic apparatus with the subject eye as reference, the left-right axis in the left-right direction (horizontal direction) is indicated by X-axis, the vertical axis in the up-down direction (vertical direction) by Y-axis, and the front-back axis in the front-back direction (depth direction) perpendicular to the X and Y axes by Z axis.

### FIRST EMBODIMENT

### [ENTIRE APPARATUS CONFIGURATION (FIGS. 1-4)]

As shown in FIGS. 1-4, the ophthalmologic apparatus A includes a pedestal portion 10, a body portion 20, a chin support portion 30, a control panel 40, an optical system 50, and a controller 60.

The ophthalmologic apparatus A includes a fundus camera that acquires fundus images of the subject eye E, and an OCT (abbreviation of Optical Coherence Tomography) that acquires fundus tomographic images of the subject eye E. Herein, the fundus camera refers to a camera that images a fundus condition of retina, optic nerve, capillaries, etc. at the back of the subject eye E and captures the resulting fundus images. The OCT refers to an optical coherence tomography that images a section of the retina existing at the fundus of the subject eye E by using interference of light and captures the resulting fundus tomographic images.

As shown in FIG. 4, the pedestal portion 10 is placed on a height-adjustable optometric table T, etc. On a top surface of the pedestal portion 10, the body portion 20 is supported to be movable in three axes directions of X-, Y- and Z-axes. At a front position of the pedestal portion 10, the chin support portion 30 is fixed. At a side surface position of the pedestal portion 10, there are provided a power switch 11, a power inlet 12, a USB terminal 13, and a LAN terminal 14. USB is an abbreviation of Universal Serial Bus, and LAN is an abbreviation of Local Area Network. The USB terminal is an external memory connection terminal for connecting thereto HDD (abbreviation of Hard Disk Drive), USB memory, etc. To the LAN terminal 14, a personal computer 16, in which a dedicated software, etc. is installed, is connected through a LAN cable 15.

As shown in FIG. 4, a power supply unit 17 and an XYZ driver 18 are built in an inner space of the pedestal portion 10. The power supply unit 17 includes the power switch 11, the power inlet 12, the USB terminal 13, and the LAN terminal 14, etc. The XYZ driver 18 is a motor actuator, which includes a motor and a motor drive circuit, for driving the body portion 20 in XYZ axes directions (three-dimensional direction), when moving the body portion 20 relative to the pedestal portion 10 in the alignment control.

The body portion 20 is installed to be movable in X-axis, Y-axis and Z-axis directions by the XYZ driver 18 relative to the pedestal portion 10 to which the chin support portion 30 is fixed. In the body portion 20, the optical system 50 and the controller 60 for examining ocular characteristics of the subject eye E or observing the anterior-ocular-segment or fundus of the subject eye E in a condition that the examinee's chin is supported on the chin support portion 30, are built in a body cover 21, which covers its entirety. The control panel 40 is disposed at a top position on the back surface of the body cover 21, as shown in FIGS. 1, 2 and 4.

As shown in FIG. 3, at a central portion on the front surface position of the body cover 21, there is an objective lens 51 of the optical system 50, which is opposed to the subject eye E. At a peripheral portion of the objective lens 51, there are an anterior-ocular-segment stereo camera 22 (one example of anterior-ocular-segment camera), a peripheral fixation light 23, and anterior-ocular-segment observation filters 24.

The anterior-ocular-segment stereo camera 22 is a camera that acquires anterior-ocular-segment images by photographing the anterior ocular segment of the examinee. This anterior-ocular-segment stereo camera 22 is composed of two right and left cameras 22a, 22b, which are disposed on both side positions of the objective lens 51 in a manner to incline their lens optical axes toward the anterior ocular segment of the subject eye E, which is the subject of the examination or observation. The right and left cameras 22a, 22b respectively acquire a right-side anterior-ocular-segment image and a left-side anterior-ocular-segment image by cutting out a part of the face of the examinee supported on the chin support portion 30 in accordance with the selection of the subject eye E and the angle of view at that time. Furthermore, the anterior-ocular-segment stereo camera 22 is composed of the two right and left cameras 22a, 22b that are disposed with a specific attachment width therebetween in the X-axis direction and their specific attachment angles. Therefore, once two of the two-dimensional pupil position information have been obtained based on the two anterior-ocular-segment images, it is possible to identify the three-dimensional coordinate position of the pupil by a calculation process using known information and known trigonometric functions.

The peripheral fixation light 23 is a fixation light that is used for fixing the line of sight of the subject eye E by turning it on, and eight of them are equidistantly disposed at an outer peripheral position of the objective lens 51. The anterior-ocular-segment observation filters 24 are filters that are used for adjusting the light intensity during the anterior-ocular-segment observation and the anterior-ocular-segment OCT. They are respectively disposed by the number of two (four in total) in a vertical line at an outer position of the right camera 22a and in a vertical line at outer position of the left camera 22b.

The chin support portion 30 is provided with an adjustable height position (position in the Y-axis direction) relative to a chin support holding portion 31 fixed to the pedestal portion 10 and supports the chin of the examinee. The chin support portion 30 includes a lifting rod 30a that is raised and lowered by a built-in chin support driver 32, a chin support base 30b that is fixed to a top end position of the lifting rod 30a, and chin support paper stop pins 30c that are provided at both side positions of the chin support base 30b. The chin support driver 32 is a motor actuator, which includes a motor and a motor drive circuit, for driving the lifting rod 30a in the Y-axis direction, when moving the chin support portion 30 in the Y-axis direction relative to the chin support holding portion 31 (= pedestal portion 10) in the alignment control.

The chin support holding portion 31 is a T-shaped object with both end positions to which a face support frame portion 33 is fixed. The face support frame portion 33 has a shape to surround the face of the examinee from three directions, when the examinee's chin is supported on the chin support portion 30. The face support frame portion 33 includes a pair of vertical frame portions each extending in the Y-axis direction and each provided with a height mark 33a that serves as a guide for the height position of the subject eye E. The face support frame portion 33 further includes a horizontal frame portion that stretches between the top ends of the vertical frame portions and that is provided with a removable forehead support portion 33b made of silicone rubber, etc. Furthermore, the face support frame portion 33 is provided, at an upper position of a center of the horizontal frame portion, with an arm 34 that can be bent in multiple steps and that is provided at its tip with an external fixation target 35.

The control panel 40 is disposed at the top position on the back surface of the body cover 21 and includes a display screen 41 that displays in color the anterior-ocular-segment images of the subject eye E from the anterior-ocular-segment stereo camera 22 and the anterior-ocular-segment observation images of the subject eye E from the optical system 50, etc. The display panel is a touch panel on which the examiner can touch the displayed button images and other images with his/her finger as an input operation to the controller 60. The control panel 40 is attached to the body portion 20 through a connecting support portion 42 with a support structure as a combination of bending support and rotary support. This support structure makes it possible to set the display screen 41 relative to body portion 20 at any position in the whole circumferential direction and to freely set the inclination angle of the display screen 41.

A remote operation tablet 40' is used in place of the control panel 40, when the examiner performs an examination of ocular characteristics, etc. by remote control from a position remote from the examinee. The remote operation tablet 40' has a touch-panel display screen 41'with input operation functions equivalent to those of the control panel 40 and has communication functions to communicate with the body portion 20.

The optical system 50 observes the subject eye E and examines ocular characteristics of the subject eye E, in a condition that the chin of the examinee is supported on the chin support portion 30. As shown in FIG. 4, it includes a fundus camera unit 52 with the objective lens 51, and an OCT unit 53. The fundus camera unit 52 includes an illumination optical system and an imaging optical system and is a unit constituting a fundus camera that acquires fundus images by lens, an image sensor, etc. The OCT unit 53 is a unit constituting the OCT that acquires fundus tomographic images of the subject eye E by a variable wavelength light source, fiber coupler, etc. The optical system 50 can acquire not only fundus images and fundus tomographic images of the subject eye E, but also anterior-ocular-segment observation images of the subject eye E. A detailed configuration of the optical system 50 is explained hereinafter.

The controller 60 controls each part (fundus camera unit 52, OCT unit 53, chin support portion 30, body portion 20, etc.) of the apparatus, based on various input operations such as touch operation to the display screen 41 of the control panel 40, etc. As shown in FIG. 4, the controller 60 includes a hardware configuration of a control board 60a, a CPU board 60b, and a graphic board 60c.

### [OPTICAL SYSTEM CONFIGURATION (FIG. 5)]

As shown in FIG. 5, the optical system 50 incudes the fundus camera unit 52 with the objective lens 51, and the OCT unit 53. The fundus camera unit 52 includes an alignment optical system 521, an imaging optical system 522, a focus optical system 523, an illumination optical system 524, and an OCT optical system 525.

The alignment optical system 521 generates an alignment bright spot by projecting an output light from an LED 521a as the light source onto an anterior ocular segment Ea (= cornea surface) and a fundus Ef of the subject eye E through the objective lens 51. That is, the output light (alignment light) from the LED 521a travels through diaphragms 521b, 521c and a relay lens 521d and is reflected by a dichroic mirror 521e. Then, it passes through an aperture part of an aperture mirror 524k, penetrates a dichroic mirror 525g, and is projected on the anterior ocular segment Ea and the fundus Ef of the subject eye E by the objective lens 51.

The imaging optical system 522 acquires anterior-ocular-segment front images and fundus front images of the subject eye E by a CCD image sensor 522a. In a condition that an imaging lens 522b is inserted into the optical path, the focus is taken on the anterior ocular segment Ea. In a condition that the imaging lens 522b is out of the optical path of the imaging optical system 522, the focus is taken on the fundus Ef. That is, the alignment light reflected by the anterior ocular segment Ea penetrates the objective lens 51 and the dichroic mirror 525g, travels through the aperture part of the aperture mirror 524k, and partly passes through the dichroic mirror 521e. Then, the light passes through a focusing lens 522e, a condenser lens 522c, and the imaging lens 522b, and is projected onto the CCD image sensor 522a. In contrast, the alignment light reflected by the fundus Ef penetrates the objective lens 51 and the dichroic mirror 525g, travels through the aperture part of the aperture mirror 524k, and partly passes through the dichroic mirror 521e. Then, the light passes through the focusing lens 522e and the condenser lens 522c, and is projected onto the CCD image sensor 522a.

The focus optical system 523 generates a target (split target) to take a fucus on the fundus Ef. When conducting the focus adjustment, a reflection surface of a reflection member 523g is arranged in a slanted position in an optical path of the illumination optical system 524. The light (focus light) output from an LED 523a of the focus optical system 523 passes a relay lens 523b, and is split into two light fluxes by a split target plate 523c. Then, the light passes through a two-hole diaphragm 523d, is reflected by a mirror 523e, and is reflected after an image is once formed on the reflection surface of the reflection member 523g by a condenser lens 523f. Further, the focus light travels through a relay lens 524j, is reflected by the aperture mirror 524k, penetrates the dichroic mirror 525g, and is refracted by the objective lens 51, thereby being projected onto the fundus Ef. The fundus reflection light of the focus light passes through a route similar to that of the fundus reflection light of the alignment light and is detected by the CCD image sensor 522a.

The illumination optical system 524 irradiates the fundus Ef with an observation illumination light. Light (observation illumination light) output from an observation light source 524a is reflected by a reflection mirror 524b with a curved reflection surface, and becomes a near-infrared light after passing through a visible cut filter 524d via a condenser lens 524c. Further, the observation illumination light is once converged near an imaging light source 524e, is reflected by a mirror 524f, and passes through a relay lens 524g, 524h, a diaphragm 524i and a relay lens 524j. Then, the observation illumination light is reflected on a peripheral part (a surrounding region of an aperture part) of an aperture mirror 524k, penetrates a dichroic mirror, and is refracted by the objective lens 51, thereby illuminating the fundus Ef. The fundus reflection light of the observation illumination light is refracted by the objective lens 51, penetrates the dichroic mirror 525g, passes through the aperture part formed in a center region of the aperture mirror 524k, and passes through the dichroic mirror 521e. The reflection light travels through a focusing lens 522e and the condenser lens 522c, and is projected on the CCD image sensor 522a. An observation image of the anterior ocular segment of the subject eye E is displayed in case that the focus of the imaging optical system 522 is taken on the anterior ocular segment Ea by inserting the imaging lens 522b.

The OCT optical system 525 guides a signal light from the OCT unit 53 to the fundus Ef via the imaging optical system 522 and forms an OCT measurement optical path to guide the reflection light from the fundus Ef to the OCT unit 53 via the imaging optical system 522. The OCT measurement light path is provided with a collimator lens unit 525a, an optical-pathlength changing portion 525b, a galvano scanner 525c, a focusing lens 525d, a mirror 525e, a relay lens 525f, and a dichroic mirror 525g. The dichroic mirror 525g splits the OCT measurement optical path from the fundus imaging optical path, thereby reflecting the light in a wavelength band used for the OCT measurement and transmitting the light for anterior-ocular-segment imaging and fundus imaging.

### [CONTROL SYSTEM CONFIGURATION (FIG. 6)]

As shown in FIG. 6, a control system of the ophthalmologic apparatus A includes the controller 60. To the controller 60, the XYZ driver 18, the anterior-ocular-segment stereo camera 22, the chin support driver 32, and the control panel 40, and the optical system 50 are connected.

The controller 60 includes a main controller 61 that controls the fundus camera unit 52 and the OCT unit 53, a storage 62 that stores necessary data, and an alignment controller 63. The alignment controller 63 conducts an alignment control to adjust a relative positional relationship between the subject eye E and the body portion 20 (objective lens 51), based on an anterior-ocular-segment image that has been acquired by the anterior-ocular-segment stereo camera 22, and an anterior-ocular-segment front image and a fundus front image that have been acquired by the optical system 50. The storage 62 includes a trained-model setting unit 621. The alignment controller 63 includes a chin support height adjuster 631, a pupil detection processing unit 632, an automatic alignment unit 633, and a manual alignment unit 634.

The alignment controller 63 acquires an anterior-ocular-segment image by respectively photographing left and right anterior-ocular-segments of the face of the examinee from two different directions by the anterior-ocular-segment stereo camera 22 (right camera 22a, left camera 22b). Furthermore, it acquires an anterior-ocular-segment front image and a fundus front image by photographing respective anterior ocular segments and fundi of the right and left eyes of the subject eye E by the optical system 50. The alignment controller 63 adjusts a relative positional relationship between the subject eye E and the objective lens 51 mounted on the body portion 20 by a drive command that is output to at least one of the XYZ driver 18 and the chin support driver 32. Herein, regarding which of the XYZ driver 18 and the chin support driver 32 is used, when the adjustment movement is only in XZ axes directions, the XYZ driver 18 is used. In contrast, when the adjustment movement contains a movement in Y-axis direction, the XYZ driver 18 and the chin support driver 32 are used separately, due to the fact that the Y-axis movable range of the chin support driver 32 is wider than that of the XYZ driver 18. For example, at the Y-axis movement, the chin support driver 32 is used for the chin support height adjustment, but the XYZ driver 18 is used for the automatic alignment.

After confirming that the subject eye E is seen in the anterior-ocular-segment image displayed on the screen, the chin support height adjuster 631 adjusts the height of the chin support portion 30 by the chin support driver 32 such that the pupil center of the subject eye E is at around the center of the anterior-ocular-segment image.

The chin support height adjustment is conducted by the examiner's manual operation on the display screen 41, while the examiner watches an anterior-ocular-segment front image and an anterior-ocular-segment image that are displayed on the control panel 40. Specifically, in the anterior-ocular-segment front image displayed on the display screen 41, the examiner conducts a tap operation on the displayed pupil such that the pupil is enclosed in a displayed frame of the anterior-ocular-segment front image. Then, in the anterior-ocular-segment image acquired by the camera closer to the photographed eye displayed on the display screen 41, the examiner conducts a touch operation on a displayed chin-support vertical movement button so as to adjust the height mark 33a of the chin support portion 30 to the height of the subject eye E by using the pupil display line as a guide.

The pupil detection processing unit 632 detects the pupil region of the subject eye E, based on a pupil-region prediction information that is obtained by an inference operation in which an anterior-ocular-segment camera image data captured by the anterior-ocular-segment stereo camera 22 is input to a trained pupil-region-prediction model. Herein, the trained pupil-region-prediction model refers to a trained model that is created by a training process in which a large number of teacher data are prepared by adding a pupil region information to anterior-ocular-segment camera image data collected in advance, and in which the teacher data are read into a selected machine learning model (AI model). The trained pupil-region-prediction model is read from the trained-model setting unit 621 of the storage 62. Herein, the reason why the trained-model setting unit 621 is provided in the storage 62 of the ophthalmologic apparatus A is that it makes an edge computing (one of network techniques in which devices of the edge processing are disposed close to the apparatus) since the automatic alignment to the pupil is processed in real time. The anterior-ocular-segment camera image data are respectively acquired from image sensors (not shown in the drawings) of the right and left cameras 22a, 22b of the anterior-ocular-segment stereo camera 22.

Herein, the creation of the trained pupil-region-prediction model is explained. Firstly, a machine learning model that is appropriate for the pupil region prediction is selected. In the first embodiment, PSPNet (abbreviation of Pyramid Scene Parsing Network) that is a pyramid analysis network model as a machine learning model is selected. Next, with the selection of PSPNet, semantic segmentation that is a method of assigning a feature-region label to each pixel is employed, thereby creating teacher data prepared by adding a pupil region information to anterior-ocular-segment camera image data by, for example, an annotation method in which human manually conducts the operation. In other words, there are prepared a large number of teacher data created by adding pupil region information to respective pixels of the anterior-ocular-segment camera image data collected in advance. In such teacher data, the real pupil region where the pupil exists in the camera image data has been added, and the presence of eyelids and eyelashes in the collected anterior-ocular-segment camera image data is ignored. The teacher data are prepared by the number (e.g., several hundreds) necessary for the target pupil-region prediction accuracy. Then, a trained pupil-region prediction model is created by a learning process in which a large number of teacher data whose data sizes have been normalized are read into the selected PSPNet.

In connection with the number of the trained pupil-region prediction models to be created, the goal is to create a single trained model that is common to various ophthalmologic apparatuses, not one model dedicated to only one type of ophthalmologic apparatus. For example, the goal is to create a single trained pupil-region prediction model, which is common to various ophthalmologic apparatuses, by collecting a large number of anterior-ocular-segment stereo camera image data regardless of the type or model of ophthalmologic apparatus, then adding the pupil region information to the anterior-ocular-segment stereo camera image data to create teacher data, and then using such teacher data. However, considering that the arrangement of the anterior-ocular-segment camera may become different by the apparatus design change, it is optional to create a trained pupil-region prediction model for each apparatus or each camera arrangement by using the anterior-ocular-segment camera image data that have been collected separately from those derived from different camera arrangements. A detailed pupil detection process in the pupil detection processing unit 632 is described hereinafter.

In the case of using the anterior-ocular-segment stereo camera 22, when the pupil detection processing unit 632 has succeeded in the pupil detection based on the camera image data from two directions, the automatic alignment unit 633 implements an automatic alignment relative to the pupil, based on the two sets of pupil center coordinates acquired by the successful pupil detection.

Herein, the implementation of the automatic alignment relative to the pupil is explained. Firstly, the three-dimensional current coordinates (xo, yo, zo) of the pupil center are calculated. Next, using the three-dimensional target coordinates (xt, yt, zt) and the calculated three-dimensional current coordinates (xo, yo, zo) of the pupil center, the X-axis difference Δx (= xt - xo), the Y-axis difference Δy (= yt - yo), and the Z-axis difference Δz (= zt - zo) are respectively calculated. Next, the drive command to move the body portion 20 is output to the XYZ driver 18, in accordance with the X-axis difference Δx, the Y-axis difference Δy and the Z-axis difference Δz. It is determined whether or not each of the X-axis difference Δx, the Y-axis difference Δy and the Z-axis difference Δz, after the movement of the body portion 20 resulting from the output of the drive command, is equal to or lower than an alignment threshold (e.g., a value around ±0.2 mm). During which it is determined that each of the X-axis difference Δx, the Y-axis difference Δy and the Z-axis difference Δz exceeds the alignment threshold, the difference calculation and the drive are repeated. When it is determined that each of the X-axis difference Δx, the Y-axis difference Δy and the Z-axis difference Δz has become equal to or lower than the alignment threshold, the automatic alignment relative to the pupil ends.

The calculation of the three-dimensional current coordinates (xo, yo, zo) of the pupil center is explained. Firstly, the two sets of pupil center coordinates acquired by the successful pupil detection are obtained from the pupil detection processing unit 632. Next, using the acquired two sets of pupil center coordinates, a known span between the attached left and right cameras, known camera attachment positions and the known setting angle of the camera lens optical axis, there is drawn, in a three-dimensional space, a triangle by connecting the current pupil center position, the left camera position, and the right camera position. Next, the three-dimensional current coordinates (xo, yo, zo) of the pupil center are calculated by using trigonometric functions for the drawn triangle. The three-dimensional target coordinates (xt, yt, zt) of the pupil center are determined in a manner that the position where the focus is taken in the Z-axis direction on the anterior ocular segment of the subject eye E is set as the Z-axis target coordinate zt of the pupil center and that the position where an optical axis OA of the objective lens 51 and the pupil center coincide with each other in an XY coordinates plane at the position of the target coordinate zt is set as the XY axes target coordinates xt, yt. In this way, the automatic alignment relative to the pupil is implemented on condition that the pupil detection from two directions for the photographed eye succeeds.

The manual alignment unit 634 implements the manual alignment control by the examiner's manual operation on the display screen 41, when the automatic alignment is not yet completed even though the elapsed time from the start has already reached the time limit, or when the examiner willingly selects the manual operation.

In the manual alignment control, if the manual mode button displayed on the automatic alignment screen is tapped, the automatic alignment to the pupil is stopped, and it moves to the manual adjustment mode to manually adjust the photographed eye. In the manual mode adjustment, a tap operation is conducted on pupil marks of the two anterior-ocular-segment images displayed on the display screen 41 of the control panel 40. By driving the XYZ driver 18 based on this tap operation, the XYZ axes alignment adjustment is conducted to overlap the two pupil marks at the center position of the anterior-ocular-segment image.

### [CAPTURING PROCESS OPERATION OF SUBJECT EYE IMAGE (FIG. 7)]

A capturing process operation, when capturing a subject eye image (for example, any of anterior-ocular-segment image, fundus image, and fundus tomographic image), to be conducted in the controller 60 is explained with reference to the flowchart shown in FIG. 7. The capturing process operation starts, after confirming the examinee by the ophthalmologic apparatus A of which power switch has been turned on.

In Step S1, following Start, a patient registration is conducted by the name or patient ID that identifies the patient (examinee). Here, the patient ID is an identification number for handing personal information related to the ophthalmologic examinations of the examinee, and may contain age, gender, previous examination information for following-up, etc.

In Step S2, following the patient registration in Step S1, the examiner adjusts the chin support height by manual operation. This chin support height adjustment is conducted by the chin support height adjuster 631 of the alignment controller 63.

In Step S3, following the chin support height adjustment in Step S2, the capturing mode is selected. This capturing mode selection is conducted by selecting any capturing mode (anterior-ocular-segment image capturing mode, fundus image capturing mode, fundus tomographic image capturing mode, etc.) by a touch operation from a capturing icon selection screen displayed on the display screen 41 of the control panel 40. Together with the selection of the capturing mode, the capturing eye is selected by a touch operation on the display button.

In Step S4, following the capturing mode selection in Step S3, the pupil detection processing to the subject eye (= capturing eye) is performed. The pupil detection processing is performed by the pupil detection processing unit 632 of the alignment controller 63.

In Step S5, following the successful pupil detection from two directions in the pupil detection processing in Step S4, the automatic alignment to the pupil is performed. The automatic alignment is performed by the automatic alignment unit 633 of the alignment controller 63. If the automatic alignment time is prolonged, or if the examiner has a willingness, the manual alignment (manual adjustment) to the pupil is conducted by the manual alignment unit 634, in place of the automatic alignment to the pupil.

In Step S6, following the automatic alignment of Step S5 or being not good (NG) in the preview checking in Step S8, automatic focusing that automatically adjusts focus is conducted. In this automatic focusing, focus is taken on the anterior ocular segment Ea of the subject eye E in the anterior-ocular-segment capturing mode, and is taken on the fundus Ef of the subject eye in the fundus image capturing mode and the fundus tomographic image capturing mode.

In Step S7, following the automatic focusing in Step S6, the subject eye image (e.g., any of the anterior-ocular-segment image, the fundus image and the fundus tomographic image) is captured. In this capture, the present capture is conducted by a tap operation on an OK button on the capture screen displayed on the display screen 41, followed by the next capture. A preview of the capture image is displayed at the time of each capture.

In Step S8, following the capture in Step S7, the result of preview of the captured image is checked to determine if it is OK or not good (NG). If determined as being OK, the process proceeds to Step S9. If determined as being not good (NG), the process returns to Step S6. In other words, if determined as being not good (NG), the process returns to Step S6 to repeat the automatic focusing. Then, it is possible to try the capture in Step S7 until determining that the result of preview checking is OK in Step S8.

In Step S9, following the determination that the result of preview checking is OK in Step S8, the captured image is stored in the storage 62, followed by proceeding to END.

### [Regarding PSPNet structure (FIG. 8)]

The PSPNet structure selected as one example of machine learning models in the pupil detection processing unit 632 is explained with reference to FIG. 8. The PSPNet is an FCN (abbreviation of Fully Convolutional Network) in which four-scale context features can be also additionally used by utilizing a spatial pyramid pooling in the final layer, thereby increasing accuracy for an input image that requires a wide-range context. The FCN is a method of using CNN for the semantic segmentation task and refers to a model constituted of only convolutional layers without using a fully connected layer.

As shown in FIG. 8, a PSPNet 80 includes an input image data 81, a CNN 82 (note that CNN is an abbreviation of Convolutional Neural Network), a feature map 83, a pyramid pooling module 84, a convolutional layer 85, and an output prediction data 86.

The CNN 82, which is called a convolutional neural network, is a network architecture for deep learning that learns directly from the input image data 81 and generates the feature map 83. In other words, when the input image data 81 is input to the CNN 82, the feature map (D channel) is generated by collecting a wide-range global surrounding information (wide-range context) for each pixel's feature vector.

The pyramid pooling module 84 generates an augmented feature map 841 by conducting the average pooling at four pyramid levels. Firstly, poolings are respectively performed on the entire image by grids of [6 × 6], [3 × 3], [2 × 2] and [1 × 1] to create four post-pooling feature maps. Next, the four post-pooling feature maps are respectively reduced in dimension from D channel of the original feature map 83 to D/4 channel by a 1 × 1 convolutional layer. Next, the four post-pooling feature maps are subject to upsampling, thereby having a uniform spatial size as that of the original feature map 83. Finally, the four post-pooling feature maps are concatenated or combined as the subsequent channels with the original feature map 83, thereby generating the augmented feature map 841. Herein, "pooling" refers to a process of reducing the image size according to rule. "Upsampling" refers to a process of increasing the spatial resolution of the feature map.

The convolutional layer 85 acquires the output prediction data 86, based on the augmented feature map 841 of the pyramid pooling module 84. In other words, when the augmented feature map 841 is input to the convolutional layer 85, class identification is performed for each pixel, thereby acquiring the final output prediction data 86.

Therefore, suppose that the input image data is a stereo camera image data and that the feature map is a pupil region, the output prediction data to be finally acquired is a model output pupil probability map. When the stereo camera image data is subdivided into respective pixels, the model output pupil probability map will be the one in which larger values for higher pupil probability levels are written per-pixel in regions with higher pupil probabilities. For example, in the output prediction data 86 (= model output pupil probability map) shown in FIG. 8, a black background indicates a region other than the pupil, in which a low pupil probability value such as zero has been written, and an open portion (circle) indicates a region in which a high probability value has been written.

### [PUPIL DETECTION PROCESSING CONFIGURATION (FIG. 9)]

A pupil detection processing configuration to be executed in the pupil detection processing unit 632 is explained with reference to the flowchart of FIG. 9. The flowchart of FIG. 9 is executed by a serial processing that performs a first pupil detection processing for one camera image of the anterior-ocular-segment stereo cameral images, and then performs a second pupil detection processing for the other camera image thereof after completion of the first pupil detection processing. Since both of the right and left camera image data are video data, one frame of still camera image acquired at the start of the pupil detection processing is used as the input image, and the processing is repeated in a predetermined controlled cycle (for example, 8 milliseconds). The pupil detection processing may be executed by a parallel processing in which the pupil detection processings for the right and left camera images are simultaneously executed.

In Step S401, following Start, an anterior-ocular-segment camera still image from one camera of the anterior-ocular-segment stereo camera 22 is acquired, followed by applying, as a preliminary processing, resizing to an input size conforming to the trained pupil-region prediction model and normalization. In the resizing, for example, the size of the acquired anterior-ocular-segment camera still image is resized to a half size to decrease resolution.

In Step S402, following the resizing/normalization in Step 401, the resized anterior-ocular-segment camera still image is input to trained pupil-region prediction model, thereby executing an inference. Using the anterior-ocular-segment camera still image and the trained pupil-region prediction model, the inference is executed, thereby acquiring the model-output pupil probability map as a model output information.

In Step S403, following the acquisition of the model-output pupil probability map in Step S402, the pupil probability values written in the model output pupil probability map are subjected to a numerical conversion processing by an activating function, thereby obtaining a pupil probability map. Herein, the activating function refers to a non-linear conversion processing function of neural network, and its typical example is sigmoid function that converts any values to values between 0.0 to 1.0. In other words, since the numerical values are written with no limit in the model-output pupil probability map output from the trained pupil-region prediction model, the width of those values becomes wide. In contrast, once those values are subjected to the numerical conversion processing by sigmoid function, the width of the pupil probability values is converted to a narrow range of from 0.0 to 1.0.

In Step S404, following the acquisition of the pupil probability map in Step S403, a pupil candidate region(s) is acquired by a threshold processing. Herein, the threshold processing refers to an extraction processing that extracts or leaves a region(s) having a pupil probability value(s) equal to or greater than a first threshold (e.g., around 0.6), as a pupil candidate region(s), in the pupil probability map in which the pupil probability values in a range of from 0.0 to 1.0 are written. In other words, it refers to a processing to delete, from the pupil probability map, a region(s) that is less than the first threshold in pupil probability value. The threshold processing to extract the pupil candidate region(s) by using the first threshold is one example of distribution identification processings of the pupil probability values in the pupil probability map.

In Step S405, following the acquisition of the pupil candidate region(s) of Step S404, labeling to assign a label(s) to the acquired pupil candidate region(s) is conducted.

In Step S406, following the labeling of Step S405, it is determined if the labeled number is plural or not. If YES (plural), the process proceeds to Step S408. If NO (singular), the process proceeds to Step S407.

In Step S407, following the determination of NO (singular) in Step S406, a pupil label is assigned to the labeled single pupil candidate region. In other words, the pupil label is assigned to the single pupil candidate region, such that the pupil candidate region to which the pupil label has been assigned is detected as being the pupil region. In Step S408, following the determination of YES (plural) in Step S406, the pupil label is assigned to the maximum label (the region area is maximum) pupil region candidate region of the labeled plural pupil candidate regions. The pupil label is assigned to the pupil candidate region with the maximum region, such that the pupil candidate region to which the pupil label has been assigned is detected as the pupil region. This process is one exemplary method in which, when the number of the pupil candidate regions is plural, the pupil candidate region with the maximum region area is determined as being a candidate region with the highest pupil probability.

In Step S409, following the detection of the pupil-label-assigned pupil region in Step S407 or Step S408, an elliptic approximation is applied to the detected pupil region to obtain the pupil center coordinates (XY coordinates) representing the position of the elliptic center point. The obtainment of the pupil center coordinates by applying the elliptic approximation is one example in which the pupil center coordinates are obtained based on the shape of the detected pupil region.

In Step S410, following the obtainment of the pupil center coordinates in Step S409, it is determined whether or not the area of the detected pupil region is within the set area range (first pupil determination condition). If it is OK (first pupil determination condition is satisfied), the process proceeds to Step S411. If it is NG (first pupil determination condition is not satisfied), the process proceeds to Step S412. Herein, for example, the set area range may have an upper limit calculated from the diameter (9.6 mm) obtained by multiplying 8 mm (pupil diameter being considered large) by 1.2, and a lower limit calculated from the diameter (1 mm) obtained by multiplying 2 mm (pupil diameter being considered small) by 0.5.

In Step S411, following the determination as being OK in Step S410, it is determined, based on the pupil probability map, whether or not the occupancy percentage of pupil probability values is equal to or higher than a set percentage, relative to the entirety of the detected pupil region (second pupil determination condition), and herein such pupil probability values are defined as being equal to or higher than a second threshold that is higher than the first threshold. If it is OK (second pupil determination condition is satisfied), the process proceeds to Step S413. If it is NG (second pupil determination condition is not satisfied), the process proceeds to Step S412. Herein, the second threshold is set at a value (e.g., around 0.8) that is higher than the first threshold (e.g., around 0.6). The set percentage is set at the occupancy percentage (e.g., around 70-80 %) of high values, based on occupancy-percentage experimental data in case that the detected pupil region is the real pupil region. The second pupil determination condition in Step S411 is an exemplary condition in which aggregation of pupil probability values that are equal to or higher than a threshold in the detected pupil region is checked by the pupil probability map.

As mentioned above, when at least one of the first pupil determination condition in Step S410 and the second pupil determination condition in Step S411 is not satisfied, the process proceeds to Step S412 in which an error setting as being indicative of a failure in the pupil region detection is made, followed by proceeding to the end.

In Step S413, following the determination as being OK in Step S411, the camera still image is resized to return to the original size, and a detection processing result is output as the pupil region detection success, after the satisfaction of both of the first and second pupil determination conditions, followed by proceeding to the end. Herein, the detection processing result refers to the pupil center coordinates to be output to the automatic alignment unit 633.

### [BACKGROUND TECHNOLOGY AND TASK (FIGS. 10 and 11)]

In the case of the ophthalmologic apparatus A, when conducting an ocular characteristics examination or photographing of a subject eye with an examinee's chin supported by a chin support, the position of the subject eye E shifts with each change of examinees. Therefore, it is necessary to conduct an alignment control to adjust a relative positional relationship between the subject eye E and the body portion 20.

However, stereo camera images captured by the anterior-ocular segment stereo camera 22 are prone to vignetting, in which the pupil is hidden due to the reflection of eyelids and eyelashes. In particular, in the case of the ophthalmologic apparatus A including the fundus camera and OCT with a wide angle of view, the lens diameter of the objective lens 51 becomes larger due to optical limitations accompanied by a trend toward a wider angle of view. Thus, the camera mounting angle of the anterior-ocular-segment stereo camera 22 that is constituted of the right and left cameras 22a, 22b disposed on both sides of the objective lens 51 becomes more acute than the camera mounting angle when the lens diameter is smaller (see FIGS. 3 and 5). This results in an elongated oval shape of the pupil of the subject eye E, and there is more likely to occur vignetting in which the pupil is hidden by the reflection of eyelids, eyelashes, etc. The right and left cameras 22a, 22b of the anterior-ocular-segment camera 22 are in fact disposed in a left-right positional relationship as shown in FIG. 3, but they are disposed in a vertical positional relationship in FIG. 5 for convenience.

In contrast, in the background technology, the pupil of the subject eye E is detected, based on the binarized image acquired by an image processing that recognizes the pupil region from the stereo camera image prepared by capturing the anterior ocular segment. The pupil detection processing configuration in this background technology is explained with reference to the flowchart of FIG. 10. The flowchart of FIG. 10 is, similar to FIG. 9, executed by a serial processing that performs one pupil detection processing for the right camera image of the anterior-ocular-segment stereo cameral images, and another pupil detection processing for the left camera image thereof. Since both of the right and left camera image data are video data, one frame of still camera image acquired at the start of the pupil detection processing is used as the input image, and the processing is repeated in a predetermined controlled cycle.

In Step S501, following the start, a 1/4 image is created by cutting the anterior-ocular-segment still camera image from the stereo camera. In the next Step S502, the 1/4 image is searched for a bright spot (= alignment bright spot) that has been projected onto the anterior ocular segment Ea of the subject eye E from the alignment optical system 521. In Step S503, it is determined whether or not the alignment bright spot exists in the 1/4 image. If the bright spot exists, the process proceeds from Step S503 to Step S504. If the bright spot does not exist, it proceeds from Step S503 to Step S505.

In Step S504, a partial image taken by cutting around the bright spot is binarized. In Step S505, the entire image is binarized. Herein, the binarization refers to a process of creating an image that is separated into low and high luminance regions according to the luminance threshold set at a low luminance to extract the pupil region.

In Step S506, following the binarization in Step S504 or Step S505, the low luminance region(s) extracted by the binarization is labeled. In the next Step S507, from the low luminance region(s) extracted by the binarization, the low luminance region(s) with a small area that is less than a set pupil area threshold is deleted.

In Step S508, there is determined the number (n) of labels assigned to the low luminance region(s) that remains after deleting the small-area low luminance region(s). If n = 0, the process proceeds to Step S509. If n > 1, it proceeds to Step S510. If n = 1, it proceeds to Step S511. In Step S509, following the determination of n = 0 in Step S508, an error setting as being indicative of a failure in the pupil region detection is made, followed by proceeding to the end.

In Step S510, following the determination of n > 1 in Step S508, the label assigned to the low luminance region that is maximum in roundness is selected from the remaining low luminance regions. Herein, "roundness" is an index value representing closeness to circle, which is determined from a relationship between the region area and perimeter, and is calculated from a formula of "Roundness = 4πS/L² (S: area; L: perimeter)". In Step S511, following the determination of n = 1 in Step S508, the low luminance region determined as n = 1 is acquired as a pupil candidate. Instead, following the label selection in Step S510, the selected low luminance region is acquired as a pupil candidate. In the next Step S512, following acquisition of the pupil candidate in Step S511, an elliptic approximation is applied to a contour edge of the pupil candidate to acquire the pupil center coordinates (XY coordinates).

In Step S513, following the elliptic approximation to the contour edge in Step S512, it is determined whether or not a pupil determination condition by shape is satisfied. If it is OK (satisfaction of the pupil determination condition), a detection processing result (pupil center coordinates) due to the pupil region detection success is output, followed by proceeding to the end. In contrast, if it is NG (no satisfaction of the pupil determination condition), the process proceeds to Step S509, an error setting as being indicative of a failure in the pupil region detection is made, followed by proceeding to the end. Herein, the pupil determination condition by shape refers to the roundness condition in which the calculated value of roundness of the pupil candidate is equal to or greater than the roundness determination threshold, and the region area condition in which the calculated value of the region area of the pupil candidate is equal to or greater than the area determination threshold. Therefore, only if both of the roundness condition and the region area condition of the pupil candidate are satisfied, the pupil detection is determined as success. If at least one of these two conditions is not satisfied, the pupil detection is determined as failure.

Next, the task of the pupil detection in the background technology is explained with reference to FIG. 11. In the background technology, as shown in (a) of FIG. 11, the input information is a stereo camera image in which an eyelid LI and eyelashes LA are reflected on an upper part of the pupil P.

The pupil region detection method in the background technology is as shown in the flowchart of FIG. 10. In other words, the stereo camera image shown in (a) of FIG. 11 is searched for the bright spot BS projected onto the anterior ocular segment Ea to detect it. If the bright spot BS is not detected, the entire image is subjected to a binarization by using a luminance threshold to distinguish the pupil P which has the lowest luminance in the image. As shown in (b) of FIG. 11, if the bright spot BS is detected, an image around the bright spot is subjected to the binarization by using the luminance threshold to distinguish the pupil P which has the lowest luminance in the image. In the method, the pupil region is recognized and detected from the image acquired by the binarization.

Therefore, in the case of the background technology in which the pupil region is detected by using the stereo camera image, as shown in (c) of FIG. 11, if there is a reflection of the eyelid LI or eyelashes LA with a luminance higher than that of the pupil P, a part of the pupil region becomes missing through the binarization, resulting in an image with an irregular pupil region shape PS. In this way, if the pupil image contains a part with a luminance level higher than that of the pupil, such high-luminance part is not recognized as the pupil region, thereby lowering the recognized pupil region in roundness and region area.

Therefore, if the pupil region shape PS is determined as having a small area due to the missing of a part of the pupil region through the binarization, the low luminance region with such small area is deleted in Step S507 of the flowchart of FIG. 10. If the number (n) of labels is determined as "n = 0" in Step S508 by this deletion, the process proceeds to Step S509. In Step S509, an error setting as being indicative of a failure in the pupil region detection is made.

In contrast, despite missing of a part of the pupil region through the binarization, if the pupil region shape PS is determined as not having the small area, the low luminance region is not deleted in Step S507 of the flowchart of FIG. 10. Then, if the number (n) of labels is determined as "n > 1" in Step S508, the process proceeds to Step S510, Step S511 and then Step S512. If the number (n) of labels is determined as "n = 1" in Step S508, the process proceeds to Step S511 and then Step S512. In Step S511, the low luminance region is acquired as a pupil candidate. In Step S512, as shown in (d) of FIG. 11, the pupil center coordinates PC are acquired by applying an elliptic approximation EA to a contour edge.

However, in Step S513 after Step S512, it is determined whether or not the pupil determination condition by shape (the roundness condition and the region area condition) is satisfied. Therefore, even if the pupil candidate has been acquired, as shown by the pupil region shape PS of (c) of FIG. 11, the lowering of the pupil candidate in roundness or region area due to the reflection results in no satisfaction of the pupil determination condition by shape. If at least one of the roundness condition and the region area condition is not satisfied in Step S513, the process proceeds to Step S509. In Step S509, an error setting as being indicative of a failure in the pupil region detection is made.

Thus, in the case of the background technology to detect pupil of the subject eye E by an image processing to recognize a stereo camera image acquired by photographing the anterior ocular segment, if the pupil region is lowered in roundness or region area by an eyelid LI or eyelashes LA reflection, etc., it is difficult to precisely and stably detect the pupil region. For diseased eyes with cataract, pupil contraction, etc. too, it is difficult to precisely and stably detect the pupil region, if the low luminance region is lowered in roundness or area by the binarization as compared with the real pupil region. Furthermore, for the automatic alignment relative to the pupil too, it is executed on condition that the pupil detection from two directions relative to the photographed eye is successful. Therefore, the task that the pupil region detection is not stable is reflected as it is, thereby lowering stability of the automatic alignment relative to the pupil.

### [PUPIL DETECTION ACTION (FIGS. 9 and 12)]

Attention was focused on a point that the pupil region can be detected precisely and stably, as a result of eliminating the influence that interferes with the pupil detection in the background technology, by employing an AI image recognition technology using a trained model for the pupil detection method in an ophthalmologic apparatus, against the task of the pupil detection in the background technology.

That is, an ophthalmologic apparatus A includes a trained-model setting unit 621 that is configured to set a trained model for the images of the anterior ocular segment of the subject eye E; and a pupil detection processing unit 632 that is configured to detect a pupil region of the subject eye E. The trained-model setting unit 621 is configured to set a trained pupil-region-prediction model that is created by a training process in which a large number of teacher data are prepared by adding a pupil region information to anterior-ocular-segment camera image data collected in advance, and in which the teacher data are read into a selected machine learning model. The pupil detection processing unit 632 is configured to detect the pupil region PA of the subject eye E, based on a pupil-region prediction information as a model output that is obtained by an inference operation in which an anterior-ocular-segment camera image data 81 captured by the anterior-ocular-segment stereo camera 22 is input to the trained pupil-region-prediction model.

In this way, the pupil region detection information derives not from the anterior-ocular-segment camera image information that is affected by interference with the pupil detection such as reflection, but from the pupil region prediction information as a model output that is obtained from the trained pupil-region-prediction model by the inference operation. Therefore, the pupil region prediction information obtained as a model output becomes information that precisely predicts the real pupil region where the pupil P exists, with no relation to the influence that interferes with the pupil detection such as reflection. Therefore, even if the pupil image has a part with a luminance level that is higher than that of the pupil luminance due to the eyelid LI or eyelashes LA reflection, etc., the real region in which the pupil P exists and in which the higher-luminance-level part is included is recognized as the pupil region (see the pupil region PA of (c) of FIG. 12). Therefore, at the pupil detection, it is possible to robustly detect the pupil region PA by the AI image recognition using the trained model.

Next, the pupil detection processing action in the first embodiment is explained with reference to FIGS. 9 and 12. In the first embodiment, for comparison with the background technology, as shown in (a) of FIG. 12, as the anterior-ocular-segment camera still image that is to be input to the trained pupil region prediction model, there is used an image similar to that of the background technology ((a) of FIG. 11) in which the eyelid LI and the eyelashes LA are reflected on an upper part of the pupil P.

According to the pupil region detection processing in the first embodiment, resizing and normalization are applied in Step S401 as a preliminary processing against the acquired anterior-ocular-segment camera still image. In the next Step S402, the inference is executed by inputting the resized anterior-ocular-segment right camera still image to the trained pupil region prediction model, thereby acquiring the model-output pupil probability map. In the next Step S403, the pupil probability values written in the model-output pupil probability map are subjected to a numerical conversion processing by an activating function, thereby obtaining a pupil probability map M. This pupil probability map M is, for example, as shown in (b) of FIG. 12, a map in which, even though the eyelid LI and the eyelashes LA are reflected on an upper part of the pupil P, high pupil probability values are expressed in the entire real pupil region with no reflection. The pupil probability map M shown in (b) of FIG. 12 represents the pupil probability values as a black-and-white image with shading.

In the next Step S404, a pupil candidate region(s) is acquired by a threshold processing. In the next Step S405, labeling to assign a label(s) to the acquired pupil candidate region(s) is conducted. In the next Step S406, it is determined if the labeled number is plural or not. If it is determined as NO (singular), the process proceeds to Step S407 where a pupil label is assigned to the labeled single pupil candidate region. In contrast, if it is determined as YES (plural), the process proceeds to Step S408 where the pupil label is assigned to the maximum-label pupil-region candidate region of the labeled plural pupil candidate regions. The pupil candidate region to which the pupil label has been assigned in Step S407 or Step S408 is detected as the pupil region. For example, as shown in (c) of FIG. 12, the pupil region PA, to which the pupil label has been assigned, almost matches with the region shape of the pupil P of the subject eye E, with no relation to the eyelid LI and the eyelashes LA reflected on an upper part of the pupil P. In other words, the pupil region PA with the pupil label, which is acquired by the pupil detection processing action in the first embodiment, results in a region shape with high roundness and large area.

In the next Step S409, an elliptic approximation is applied to the pupil region PA with the pupil label, thereby obtaining the pupil center coordinates PC (XY coordinates). For example, as shown in (d) of FIG. 12, the pupil center coordinates PC are obtained as position coordinates of the ellipse center point by applying an elliptic approximation EA to the pupil region PA with the pupil label.

In the next Step S410, there is determined the first pupil determination condition whether or not the region area of the pupil region PA with the pupil label is within the set area range. If it is NG (first pupil determination condition is not satisfied), the process proceeds to Step S412 in which an error setting as being indicative of a failure in the pupil region detection is made, followed by proceeding to the end. If it is OK (first pupil determination condition is satisfied), the process proceeds to Step S411. Herein, the first pupil determination condition is a region area checking condition to exclude the case in which the region area of the pupil region PA with the pupil label results in an unrealistically small or large area due to misdetection, etc. Therefore, the first pupil determination condition is determined as OK, as long as the pupil region PA is predicted based on the pupil probability map M properly acquired.

In the next Step S411, there is determined the second pupil determination condition whether or not the occupancy percentage of a region, in which the pupil probability values are equal to or higher than the second threshold that is higher than the first threshold, relative to the pupil candidate region, which is equal to or higher than the first threshold, of the pupil probability map M, is equal to or higher than the pupil determination percentage threshold. If it is determined as NG (second pupil determination condition is not satisfied), the process proceeds to Step S412 in which an error setting as being indicative of a failure in the pupil region detection is made, followed by proceeding to the end. If it is determined as OK (second pupil determination condition is satisfied), the process proceeds to Step S413 in which the camera still image is resized to return to the original size, and in which a detection processing result is output as the pupil region detection success, followed by proceeding to the end. Herein, the second pupil determination condition is a prediction precision checking condition to check that the prediction precision of the detected pupil region PA is high, based on the distribution condition of the pupil probability values of the pupil region PA that is predicted based on the pupil probability map. Therefore, the second pupil determination condition is determined as OK, as long as the pupil region PA is predicted based on the pupil probability map M properly acquired.

Thus, in the case of the first embodiment to detect the pupil region PA based on the model-output pupil probability map, it is possible to precisely and stably detect the pupil region, due to no reflection influence in spite of the reflection of the eyelid LI and the eyelashes LA on the anterior-ocular-segment camera still image to be input, etc. In other words, precision and stability at the pupil region detection are acquired by adopting an AI image recognition using a trained pupil-region prediction model, which is capable of eliminating the influence that interferes with the pupil detection, as a pupil region PA detection method. In particular, precision at the pupil region detection is improved by including the satisfaction of the first pupil determination condition (region area checking condition) and the satisfaction of the second pupil determination condition (prediction precision checking condition) relative to the predicted pupil region PA, as the pupil region PA detection success conditions.

For diseased eyes with cataract, pupil contraction, etc. too, adopting an AI image recognition using a trained pupil-region prediction model is not affected by the shape irregularity and the area narrowing of the low luminance region in the background technology. Therefore, it is possible to precisely and stably detect the pupil region PA. Furthermore, for the automatic alignment relative to the pupil too, even if it is on condition that the pupil detection from two directions relative to the photographed eye is successful, the merit of the pupil detection processing that the pupil region PA can be stably detected from two directions is reflected, thereby improving stability of the automatic alignment relative to the pupil P.

As explained as above, the ophthalmologic apparatus A of the first embodiment brings about the following advantageous effects.
(1) An ophthalmologic apparatus A includes an anterior-ocular-segment camera (anterior-ocular-segment stereo camera 22) that captures images of an anterior ocular segment Ea of a subject eye E; a trained-model setting unit 621 that is configured to set a trained model for the images of the anterior ocular segment Ea of the subject eye E; and a pupil detection processing unit 632 that is configured to detect a pupil region PA of the subject eye E. The trained-model setting unit 621 is configured to set a trained pupil-region-prediction model that is created by a training process in which a large number of teacher data are prepared by adding a pupil region information to anterior-ocular-segment camera image data collected in advance, and in which the teacher data are read into a selected machine learning model 80. The pupil detection processing unit 632 is configured to detect the pupil region PA of the subject eye E, based on a pupil-region prediction information as a model output 86 that is obtained by an inference operation in which an anterior-ocular-segment camera image data 81 captured by the anterior-ocular-segment camera (anterior-ocular-segment stereo camera 22) is input to the trained pupil-region-prediction model. Therefore, at the pupil detection, the pupil region PA can be robustly detected by AI image recognition using the trained model.
(2) The trained-model setting unit 621 is configured to set the trained pupil-region-prediction model that is created by collecting the anterior-ocular-segment camera image data by a large number regardless of a type or model of the ophthalmologic apparatus A, then preparing the teacher data by adding the pupil region information to the anterior-ocular-segment camera image data, and then using the teacher data. Therefore, it is possible to detect the pupil region PA by the inference operation that captures characteristics of the anterior-ocular-segment camera image data 81 to be input, regardless of a type or model of the ophthalmologic apparatus A, by aiming setting one trained common model as the trained pupil-region-prediction model. Herein, the characteristics of the anterior-ocular-segment camera image data 81 refer to respective shape characteristics of the subject eye E, eyelid LI, eyelashes LA, etc., which are reflected in the frame as the anterior-ocular-segment camera image, and characteristics of positional relationship of each part relative to the entire frame.
(3) The pupil detection processing unit 632 is configured such that the inference operation by inputting the anterior-ocular-segment camera image data as a model input is conducted, thereby obtaining a pupil probability map M in which pupil probability values are written in respective pixels of the anterior-ocular-segment camera image data, and that a high-probability-value region extracted by a distribution identification processing (thresholding for binarization) of the pupil probability values in the pupil probability map M is detected as a pupil candidate region. Therefore, it is possible to detect an appropriate region with a high pupil probability value as the pupil candidate region by the distribution identification processing of the pupil probability values to extract the high-probability-value region from the pupil probability map M obtained by the inference operation when detecting the pupil candidate region.
(4) The pupil detection processing unit 632 is configured such that, when the pupil candidate region has been detected by a plural number, each pupil candidate region is labeled, then the pupil candidate region (candidate region with the maximum area) that is highest in pupil probability is provided with a pupil label, and then the pupil candidate region provided with the pupil label is detected as the pupil region PA. Therefore, it is possible to detect an appropriate region as the pupil region PA by a process in which, when the pupil candidate region has been detected by a large number, the pupil candidate region that is highest in pupil probability is provided with a pupil label and is selected as the pupil candidate region provided with the pupil label.
(5) Once the pupil region PA is detected, the pupil detection processing unit 632 is configured to obtain pupil position coordinates (pupil center coordinates PC) based on a shape of the detected pupil region PA. Therefore, it is possible to obtain the pupil position coordinates that are output as the pupil position information by a process in which, when obtaining the pupil position coordinates (pupil center coordinates PC), the pupil position is identified based on the shape of the detected pupil region PA.
(6) The pupil detection processing unit 632 is configured to determine a first pupil determination condition in which an area of the detected pupil region PA is confirmed to be within a set area range, and a second pupil determination condition in which an aggregation of the pupil probability values that is higher than a threshold in the detected pupil region PA is confirmed by the pupil probability map M. The pupil detection processing unit 632 is configured such that, when at least one of the first and second determination conditions is not satisfied, an error setting as being indicative of a failure in the pupil region detection is made, and that, when both of the first and second determination conditions are satisfied, a detection processing result as being a success in the pupil region detection is output. Therefore, it is possible to improve the certainty of detecting the pupil region PA by determining the satisfaction of both the region area confirmation condition and the prediction accuracy confirmation condition as being the condition of success in detecting the pupil region PA.
(7) The ophthalmologic apparatus A further includes a body portion 20 where an optical system 50 is built in, a driver (XYZ driver 18) that moves the body portion 20 relative to a pedestal portion 10 in a three-dimensional direction, and a controller 60 that is configured to control each part of the apparatus A. The anterior-ocular-segment camera (anterior-ocular-segment stereo camera 22) includes at least two cameras (right camera 22a, left camera 22b) that are provided at an outer peripheral position of an objective lens 51 mounted on the body portion 20 and that are provided with respective lens optical axes each inclined toward the anterior ocular segment Ea of the subject eye E. The controller 60 includes an alignment controller 63 that is configured to control an adjustment of a relative positional relationship between the subject eye E and the body portion 20, the alignment controller 63 including the pupil detection processing unit 632 and an automatic alignment unit 633 that executes an automatic alignment relative to a pupil P of the subject eye E. The pupil detection processing unit 632 is configured to detect the pupil region PA by using the anterior-ocular-segment camera image data 81 and the trained pupil-region-prediction model set by the trained-model setting unit 621. The automatic alignment unit 633 is configured, in the automatic alignment relative to the pupil P, to obtain two sets of pupil position coordinates (pupil center coordinates PC) after a success in the pupil region detection relative to the subject eye E and then to output, to the driver (XYZ driver 18), a movement command to converge three-dimensional current coordinates calculated based on the two sets of pupil position coordinates PC, on three-dimensional target coordinates of the pupil P. Therefore, even though the images from the anterior-ocular-segment cameras, which are prone to reflection, are used at the automatic alignment relative to the pupil P, it is possible to reflect a merit of the pupil detection process that is capable of stably detecting the pupil region PA, thereby improving stability of the automatic alignment.

### SECOND EMBODIMENT

The second embodiment is an example using an anterior-ocular-segment three-way camera 22' as the anterior-ocular-segment camera, in contrast with the first embodiment using the anterior-ocular-segment camera 22.

As shown in FIG. 13, the anterior-ocular-segment three-way camera 22' includes a right camera 22a, a left camera 22b and a lower camera 22c, which are provided, as constituent parts of an objective lens unit 70, on an objective lens unit case 71. Furthermore, the objective lens unit 70 includes a right-camera's photographing light 25a, a left-camera's photographing light 25b, and lower-camera's photographing lights 25c, as a camera's photographing light 25 attached to the anterior-ocular-segment three-way camera 22'. Herein, the objective lens unit 70 refers to a unit constituted by integrally attaching the anterior-ocular-segment three-way camera 22' and the camera's photographing light 25 to the objective lens 51 of the optical system 50.

The anterior-ocular-segment three-way camera 22' is configured such that, when a horizontal division line HL is drawn in a manner to pass through an optical axis OA of the objective lens 51 and to divide the lens into two halves in the vertical direction, the three cameras 22a, 22b, 22c are disposed at an outer peripheral position of the objective lens 51 and in a region below the horizontal division line HL. The camera's photographing light 25 includes the single right-camera's photographing light 25a at a position above the right camera 22a, the single left-camera's photographing light 25b at a position above the left camera 22b, and the two lower-camera's photographing lights 25c at both side positions of the lower camera 22c.

Herein, as the anterior-ocular-segment three-way camera 22', there is used a small-size camera including a lens unit and an image sensor. For the camera's photographing light 25, there is used an infrared LED (abbreviation of Light Emitting Diode) which emits infrared light toward the subject eye E to enlarge the luminance difference between the pupil P and other parts in the photographed anterior-ocular-segment image.

When a vertical division line VL is drawn in a manner to pass through the optical axis OA of the objective lens 51 and to divide the lens into two halves in the horizontal direction, the right and left cameras 22a, 22b are disposed at both side positions in line symmetry with respect to the vertical division line VL. The lower camera 22c is disposed on the vertical division line VL. The right, left and lower cameras 22a, 22b, 22c are disposed to have upward inclination angles such that their respective camera lens axes are directed toward the pupil P of the subject eye E.

The automatic alignment action relative to the pupil P is explained. In the case of the second embodiment, due to the use of the anterior-ocular-segment three-way camera 22', camera image data from the three cameras of the right, left and lower cameras 22a, 22b, 22c are acquired. Therefore, in the case of succeeding the pupil detection by all the camera image data of the three cameras 22a, 22b, 22c, at the automatic alignment relative to the pupil P, the two cameras are automatically selected, and, based on the pupil positional coordinates from the selected two cameras, the three-dimensional current coordinates of the pupil P are calculated. Herein, the method of automatically selecting the two cameras is, for example, a method of eliminating one of the three that has been determined as being lowest in roundness and probability values as the selection standards.

In the case of the second embodiment, due to the use of the anterior-ocular-segment three-way camera 22', even if the pupil detection by the camera image data from one of the three cameras 22a, 22b, 22c fails, such failure in the pupil detection is permitted. In other words, if the pupil detection by the two-cameras image data succeeds, similar to the first embodiment, at the automatic alignment relative to the pupil P, it is possible to calculate the three-dimensional current coordinates of the pupil P based on two sets of the pupil positional coordinates. Since other configurations and advantageous effects of the second embodiment are similar to those of the first embodiment, their drawings and explanations are omitted.

As above, the ophthalmologic apparatus according to the present disclosure has been described based on the first and second embodiments. However, specific configurations are not limited to those of these embodiments. Changes and additions in design should be allowed as long as they do not deviate from the gist of the inventions recited in the claims.

The first embodiment showed, so as to conform to the automatic alignment relative to the pupil P, an example of the anterior-ocular-segment stereo camera 22, as an anterior-ocular-segment camera, which is constituted of the right and left cameras 22a, 22b for photographing the anterior ocular segment of a single photographing eye from right and left directions. The second embodiment showed an example of the anterior-ocular-segment three-way camera 22' by the right, left and lower cameras 22a, 22b, 22c for photographing the anterior ocular segment of a single photographing eye from right, left and lower directions. However, the anterior-ocular-segment is not limited to the anterior-ocular-segment stereo camera or the anterior-ocular-segment three-way camera. For example, it may be constituted of at least four cameras provided at an outer peripheral position of the objective lens mounted on the body portion. Furthermore, in the case of applying the pupil detection processing of the present disclosure to various uses other than the automatic alignment, in its adaptation to each use, for example, it may be a single anterior-ocular-segment camera for photographing the anterior ocular segment from one direction relative to a single photographing eye. Furthermore, it may be an anterior-ocular-segment camera for simultaneously photographing the right-eye's anterior ocular segment and the left-eye's anterior ocular segment relative to respective photographing eyes of the right and left eyes.

The first embodiment showed a built-in example in which the trained-model setting unit 621 is installed in the storage 62 possessed by the controller 60 of the ophthalmologic apparatus A. However, a section to install the trained-model setting unit is not limited to the built-in example in which it is installed in the controller of the ophthalmologic apparatus. For example, even though it is a built-in example, it may be one in which an exclusive storage only for storing and setting the trained model is installed in a section other than the controller of the ophthalmologic apparatus. In summary, it suffices to make its installment in a section capable of conducting an edge processing by an edge computing that can process the automatic alignment relative to the pupil in real time. Therefore, as long as it is a section capable of satisfying the edge processing, the trained-model setting unit may be installed in an external memory that is connected to the external memory connection terminal.

The first embodiment showed, as the trained-model setting unit 621, an example of which goal is to set a single trained common model by using teacher data created by adding the pupil region information to a large number of anterior-ocular-segment stereo camera image data collected, regardless of the type or model of ophthalmologic apparatus. However, as the trained-model setting unit, it may be an example in which teacher data are created by collecting the anterior-ocular-segment camera image data separately from those derived from different camera arrangements and then adding the pupil region information to the collected anterior-ocular-segment camera image data, and in which a trained pupil-region prediction model created for each apparatus or each camera arrangement by using the teacher data is set. In this case, when detecting the pupil region, it is possible to deal with the situation that the characteristics of the anterior-ocular-segment camera image data become different by the change of the anterior-ocular-segment camera arrangement. As a result, it is possible to precisely detect the pupil region, regardless of the difference in camera arrangement. Furthermore, it may be an example in which the camera arrangements are roughly classified into categories, and a trained pupil-region prediction model created for each classified camera arrangement category is set.

The first embodiment showed an example in which PSPNet is selected as the machine learning model, and a trained pupil-region prediction model created by a training process to read a large number of prepared teacher data into PSPNet is set in the trained-model setting unit 621. However, the machine learning model is not limited to PSPNet. For example, UNet may be selected as the machine learning model. UNet is one of FCNs and is a convolutional neural network model developed for biomedical image segmentation. In UNet, when the decoder side conducts an expansion processing of the feature map, the feature map on the encoder side is cut out for its use to have the same size. Furthermore, as the machine learning model, it is optional to select a model other than PSPNet and UNet. In particular, there is preferable a machine learning model having a convolutional neural network structure (CNN structure) that is a network architecture for deep learning and is good at image recognition to recognize feature sections from a camera image.

The first embodiment showed an example in which the pupil detection processing unit 632 detects, as a pupil candidate region, a high-probability-value region extracted by a threshold process to subject pupil probability values in the pupil probability map M to binarization by the first threshold. However, the pupil detection processing unit is not limited to the threshold process example by binarization, as long as it is an example in which a high-probability-value region extracted by a distribution identification processing of pupil probability values in the pupil probability map is detected as a pupil candidate region. For example, it may be an example in which a plurality of pupil candidate regions are extracted by using a plurality of thresholds, and then a pupil-probability-values distribution analysis is comprehensively conducted on the plurality of pupil candidate regions to extract a pupil candidate region.

The first embodiment showed an example in which, when a plurality of pupil candidate regions have been detected, the pupil detection processing unit 632 assigns labels to respective candidate regions, and then assigns the pupil label to the candidate region that is maximum in the pupil candidate region's area. However, the pupil detection processing unit is not limited to the example that assigns the pupil label to the candidate region that is maximum in the pupil candidate region's area, as long as it is an example that can assign the pupil label to the candidate region that is highest in pupil probability. For example, it may be an example that assigns the pupil label to the candidate region that is highest in the average of the pupil probability values obtained, as model outputs, from the pupil candidate regions. Furthermore, it may be an example that assigns the pupil label to the candidate region having the maximum value of the pupil probability values obtained, as model outputs, from the pupil candidate regions.

The first embodiment showed an example in which, when the pupil region PA has been detected, the pupil detection processing unit 632 applies an elliptic approximation EA to the shape of the detected pupil region PA to acquire pupil center coordinates PC (center coordinates of the approximate ellipse). However, it is not limited to an example that acquires the pupil center coordinates, as long as the pupil detection processing unit can acquire the pupil position coordinates based on the shape of the pupil region. For example, it may be an example that acquires pupil center-of-gravity coordinates (coordinates of the position at which the shape balance is maintained).

The first embodiment showed an example in which the pupil detection processing unit 632 sets, as the second pupil determination condition, that the occupancy percentage of pupil probability values, which are defined as being equal to or higher than the second threshold that is higher than the first threshold, is equal to or higher than a set percentage, relative to the entirety of the pupil region PA. However, it is not limited to the example of the first embodiment, as long as it is an example in which the pupil detection processing unit sets, as the second pupil determination condition, a condition in which an aggregation of the pupil probability values that is higher than a threshold in the detected pupil region is confirmed by the pupil probability map. For example, it may be an example in which the second pupil determination condition is set as that the average of the pupil probability values of the entirety of the pupil region is calculated, and this average is equal to or higher than an aggregation determination value that is indicative of aggregation of high pupil probability values. Furthermore, it may be an example in which distribution of the pupil probability values of the entirety of the pupil region is determined, and it is determined if the distribution mode is a mode that is indicative of aggregation of high pupil probability values.

The first embodiment showed an example in which, in the automatic alignment relative to the pupil, after a success in the pupil region PA detection from two directions relative to the subject eye E, the automatic alignment unit 633 calculates three-dimensional current coordinates of the pupil P based on the two sets of pupil center coordinates PC. The second embodiment showed an example in which, after a success in the pupil region PA detection from three directions relative to the subject eye E, the automatic alignment unit 633 calculates three-dimensional current coordinates of the pupil P based on the two sets of pupil center coordinates PC automatically selected. However, the automatic alignment unit is not limited to the pupil region detection success from two or three directions. In other words, the calculation of the three-dimensional current coordinates requires the pupil position information at least from two directions. Therefore, in the case of the pupil detection success with camera images from four or more directions, it may be an example in which two of the cameras of success directions are automatically selected.

The first embodiment showed an example of the application to an ophthalmologic apparatus that executes an automatic alignment relative to the pupil P by using a pupil detection processing technology by AI image recognition and that includes a wide-angle fundus camera and OCT. However, the application of the ophthalmologic apparatus using the pupil detection processing technology of the present disclosure is not limited to an example of the application to an ophthalmologic apparatus including a wide-angle fundus camera and OCT. For example, as long as it is an ophthalmologic apparatus that requires execution of an automatic alignment relative to the pupil prior to observation or ocular characteristics examination of the subject eye, the application to various ophthalmologic apparatuses other than that of the first embodiment is possible.

The pupil detection processing technology according to the present disclosure repeatedly detects the pupil region in a predetermined controlled cycle by using the anterior-ocular-segment camera image data (video data) as the input image. Therefore, plotting the acquired pupil position coordinates along the time axis results in the visual-line movement tracks (eye tracking) of the subject eye. Therefore, for example, as one used for detecting the visual-line movement, it is possible to apply the pupil detection processing technology of the present disclosure to an ophthalmologic apparatus that conducts a visual-line movement examination on how the pupil position of the subject eye (left or right eye) moves when the target, on which the examinee focuses, is moved as specified. Furthermore, for example, as one used for detecting the visual-line direction of the examinee, it is possible to apply the pupil detection processing technology of the present disclosure to an ophthalmologic apparatus that conducts various subjective examinations by the displayed subjective examination screen, while the examinee wears a head-mounted display and while the visual-line direction of the examinee is monitored.

## Claims

1. An ophthalmologic apparatus (A) comprising:
an anterior-ocular-segment camera (22, 22', 22a, 22b, 22c) that captures images of an anterior ocular segment (Ea) of a subject eye (E);
a trained-model setting unit (621) that is configured to set a trained model for the images of the anterior ocular segment (Ea) of the subject eye (E); and
a pupil detection processing unit (632) that is configured to detect a pupil region (PA) of the subject eye (E),
wherein the trained-model setting unit (621) is configured to set a trained pupil-region-prediction model that is created by a training process in which a large number of teacher data are prepared by adding a pupil region information to anterior-ocular-segment camera image data collected in advance, and in which the teacher data are read into a selected machine learning model (80), and
wherein the pupil detection processing unit (632) is configured to detect the pupil region (PA) of the subject eye (E), based on a pupil-region prediction information as a model output (86) that is obtained by an inference operation in which an anterior-ocular-segment camera image data (81) captured by the anterior-ocular-segment camera (22, 22', 22a, 22b, 22c) is input to the trained pupil-region-prediction model.

2. The ophthalmologic apparatus (A) according to claim 1, wherein the trained-model setting unit (621) is configured to set the trained pupil-region-prediction model that is created by collecting the anterior-ocular-segment camera image data by a large number regardless of a type or model of the ophthalmologic apparatus (A), then preparing the teacher data by adding the pupil region information to the anterior-ocular-segment camera image data, and then using the teacher data.

3. The ophthalmologic apparatus (A) according to claim 2, wherein the pupil detection processing unit (632) is configured such that the inference operation by inputting the anterior-ocular-segment camera image data as a model input is conducted, thereby obtaining a pupil probability map (M) in which pupil probability values are written in respective pixels of the anterior-ocular-segment camera image data, and that a high-probability-value region extracted by a distribution identification processing of the pupil probability values in the pupil probability map (M) is detected as a pupil candidate region.

4. The ophthalmologic apparatus (A) according to claim 3, wherein the pupil detection processing unit (632) is configured such that, when the pupil candidate region has been detected by a plural number, each pupil candidate region is labeled, then the pupil candidate region that is highest in pupil probability is provided with a pupil label, and then the pupil candidate region provided with the pupil label is detected as the pupil region (PA).

5. The ophthalmologic apparatus (A) according to claim 4, wherein, once the pupil region (PA) is detected, the pupil detection processing unit (632) is configured to obtain pupil position coordinates (PC) based on a shape of the detected pupil region (PA).

6. The ophthalmologic apparatus (A) according to claim 5, wherein the pupil detection processing unit (632) is configured to determine a first pupil determination condition in which an area of the detected pupil region (PA) is confirmed to be within a set area range, and a second pupil determination condition in which an aggregation of the pupil probability values that is higher than a threshold in the detected pupil region (PA) is confirmed by the pupil probability map (M), and
wherein the pupil detection processing unit (632) is configured such that, when at least one of the first and second determination conditions is not satisfied, an error setting as being indicative of a failure in the pupil region detection is made, and that, when both of the first and second determination conditions are satisfied, a detection processing result as being a success in the pupil region detection is output.

7. The ophthalmologic apparatus (A) according to any one of claims 1 to 6, further comprising a body portion (20) where an optical system (50) is built in, a driver (18) that moves the body portion (20) relative to a pedestal portion (10) in a three-dimensional direction, and a controller (60) that is configured to control each part of the apparatus (A),
wherein the anterior-ocular-segment camera (22, 22', 22a, 22b, 22c) comprises at least two cameras (22a, 22b, 22c) that are provided at an outer peripheral position of an objective lens (51) mounted on the body portion (20) and that are provided with respective lens optical axes each inclined toward the anterior ocular segment (Ea) of the subject eye (E),
wherein the controller (60) comprises an alignment controller (63) that is configured to control an adjustment of a relative positional relationship between the subject eye (E) and the body portion (20), the alignment controller (63) comprising the pupil detection processing unit (632) and an automatic alignment unit (633) that executes an automatic alignment relative to a pupil (P) of the subject eye (E),
wherein the pupil detection processing unit (632) is configured to detect the pupil region (PA) by using the anterior-ocular-segment camera image data (81) and the trained pupil-region-prediction model set by the trained-model setting unit (621), and
wherein the automatic alignment unit (633) is configured, in the automatic alignment relative to the pupil (P), to obtain two sets of pupil position coordinates (PC) after a success in the pupil region detection relative to the subject eye (E) and then to output, to the driver (18), a movement command to converge three-dimensional current coordinates calculated based on the two sets of pupil position coordinates (PC), on three-dimensional target coordinates of the pupil (P).
